(19) Europäisches Patentamt
European Patent Office
Office européen des brevets

(11) Veröffentlichungsnummer: **0 360 181**
**A2**

# (12) EUROPÄISCHE PATENTANMELDUNG

(21) Anmeldenummer: **89117188.6**

(22) Anmeldetag: **16.09.89**

(51) Int. Cl.[5]: **C07F 9/30 , C07F 9/50 , A01N 57/20 , C07C 217/08 , C07C 215/10 , C07C 215/14 , C07K 5/08**

(30) Priorität: **22.09.88 DE 3832147**

(43) Veröffentlichungstag der Anmeldung: **28.03.90 Patentblatt 90/13**

(84) Benannte Vertragsstaaten: **AT BE CH DE ES FR GB GR IT LI NL**

(71) Anmelder: **HOECHST AKTIENGESELLSCHAFT**
**Postfach 80 03 20**
**D-6230 Frankfurt am Main 80(DE)**

(72) Erfinder: **Bieringer, Hermann, Dr.**
**Eichenweg 26**
**D-6239 Eppstein/Taunus(DE)**
Erfinder: **Albrecht, Konrad, Dr.**
**Sodener Strasse 64**
**D-6233 Kelkheim(Taunus)(DE)**
Erfinder: **Heinrich, Rudolf, Dr.**
**Taunusstrasse 19**
**D-6233 Kelkheim(Taunus)(DE)**
Erfinder: **Kocur, Jean, Dr.**
**Am Heiligenstock 1**
**D-6238 Hofheim am Taunus(DE)**
Erfinder: **Langelüddeke, Peter, Dr.**
**Nelkenweg 5**
**D-6238 Hofheim am Taunus(DE)**

## (54) Salze von Herbizid-Säuren mit langkettigen Stickstoffbasen.

(57) Herbizide mit verbesserter Wirkung sind Salze, welche als Säurekomponenten Herbizid-Säuren aus der Gruppe substituierte Phenoxycarbonsäuren, Phosphinsäuren der Formel

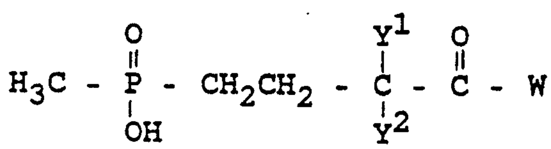

.

worin,
$Y^1$ H und $Y^2$ Amino oder $Y^1$ und $Y^2$ zusammen ein Sauerstoffatom bedeuten und
W Hydroxy oder ein von natürlichen Aminosäuren abgeleiteter Mono- oder Dipeptidrest ist,
und einer Phosphinylcarbonsäure der Formel

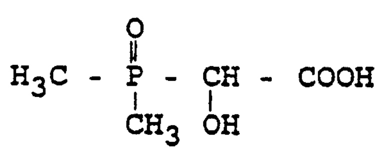

,

und als Basenkomponente ein primäres Fettamin oder Fettamid, ein sekundäres oder tertiäres Amin, ein Polyamin, ein alkyliertes, gegebenenfalls oxalkyliertes Imidazolin-Derivat, ein Zuckeramin, ein ggf. oxalkyliertes Aminoethylethanolamin, ein ggf. oxalkyliertes Alkanolamin, 1,4-Diazacyclohexan, Piperidin, Morpholin

EP 0 360 181 A2

oder Aminoxid enthalten.

## Salze von Herbizid-Säuren mit langkettigen Stickstoffbasen

Die vorliegende Erfindung betrifft neue Salze von bestimmten Herbizid-Wirkstoffen, deren Säurekomponente die wirksame Komponente darstellt. Solche Salze leiten sich ab von den Wirkstoffsäuren Glufosinate oder von anderen phosphorhaltigen Säuren oder den substituierten Phenoxycarbonsäuren wie MCPA, CMPP oder 2,4-D.

Bei der Suche nach Salzen mit überlegenen Anwendungseigenschaften wurde gefunden, daß Salze der genannten Herbizid-Wirkstoffe mit bestimmten langkettigen organischen Stickstoffbasen eine besonders intensive Herbizidwirkung entfalten, die gegenüber den bekannten Salzen deutlich erhöht ist.

Gegenstand der Erfindung sind daher neue Salze von bekannten Herbiziden aus der Gruppe, bestehend aus substituierten Phenoxycarbonsäuren, Phosphinsäuren der Formel

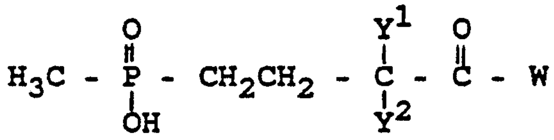

worin
$Y^1$ Wasserstoff und $Y^2$ Amino oder $Y^1$ und $Y^2$ zusammen ein Sauerstoffatom bedeuten und
W Hydroxy oder ein von natürlichen Aminosäuren abgeleiteter Mono- oder Dipeptidrest ist, und einer Phosphinylcarbonsäure der Formel

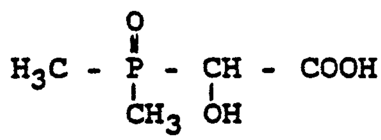

dadurch gekennzeichnet, daß sie als Basenkomponente enthalten:

a) ein primäres Fettamin oder Fettamid der Formeln (Ia) oder (Ib)

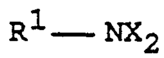

(Ia)

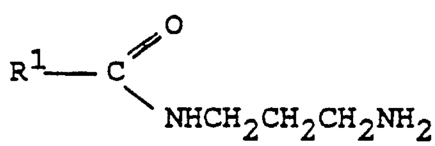

(Ib)

worin
$R^1$ = ein gesättigter oder ein- oder mehrfach ungesättigter Rest mit 6 - 22 C-Atomen, bevorzugt 12 - 14 oder 16 - 18 C-Atomen;
Alkyl- oder Alkenylphenyl, Alkyl- oder Alkenylcyclohexyl, wobei der Alkyl- oder Alkenylrest jeweils 1 - 30 C-Atome, bevorzugt 8 - 12 C-Atome enthält, und
X = Wasserstoff, Polyoxethylen mit 1 - 40 Mol EO, bevorzugt 2 - 20 Mol EO (EO = Ethylenoxid-Einheit), insbesondere Polyoxyethylen mit 2 bis 15 Mol EO, oder
Polyoxypropylen mit 1 - 45 Mol PyO, bevorzugt 2 - 15 Mol PyO (PyO = Propylenoxid-Einheit), oder ein Polyoxypropylen/Polyoxyethylen-Blockcopolymerisat im folgenden "Blockpolymerisat A" genannt, mit einem Molverhältnis PyO/EO von 1:9 bis 9:1, bevorzugt 1:1, 2:1 oder 1:2,
bedeutet;

b) ein sekundäres oder tertiäres Amin der Formeln (IIa), (IIb) oder (IIc),

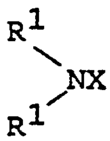

(IIa)

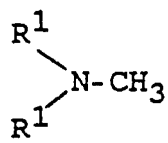

(IIb)

$R^1-N(CH_3)_2$

(IIc)

worin
$R^1$ die obengenannte Bedeutung besitzt,

c) ein Polyamin der nachfolgend unter $c_1$) - $c_4$) definierten Formeln (IIIa), (IIIb), (IIIc) oder (IIId)

$c_1$) $R^2-A-N[(CH_2)_r-N<]_m[(CH_2)_r-NX_2]_n$ (IIIa)

worin
$R^2$ = ein gesättigter oder ein- oder mehrfach ungesättigter aliphatischer Rest mit 1 - 21 C-Atomen, bevorzugt 1 - 3, 11 - 13 oder 15 - 17 C-Atomen,
A = -CO- oder $-CH_2-$, insbesondere $-CH_2-$,
m = die Zahl 0, 2, 6 oder 14, vorzugsweise 0, 2 oder 6, insbesondere 0,
n = m + 2, und
r = 2 oder 3, bevorzugt 3, bedeutet,

$c_2$) $R^2-A-NZ^1-[(CH_2)_r(NZ^1)]_s-(CH_2)_r N(Z^1)_2$ (IIIb)

worin
s = eine ganze Zahl von 1 - 20, vorzugsweise 1 bis 4
$Z^1$ = Wasserstoff oder Polyoxethylen mit 4 - 70 EO, bevorzugt 8 - 20 EO, Polyoxypropylen mit 4 - 60 PyO, bevorzugt 8 - 15 PyO oder ein Blockpolymerisat A, bedeutet,

$c_3$) $(Z^2)_2N-[(CH_2)_p-NZ^2]_o-(CH_2)_p-N(Z^2)_2$ (IIIc)

worin
o = eine ganze Zahl von 0 bis 4,
p = unabhängig voneinander eine ganze Zahl von 2 bis 6, bevorzugt 2 - 3,
$Z^2$ = Wasserstoff, Polyoxyethylen mit 5 - 70 EO, bevorzugt 8 - 25 EO, Polyoxypropylen mit 4 - 60 PyO, bevorzugt 8 - 30 PyO oder ein Blockpolymerisat A bedeutet, (hierzu zählt beispielsweise die Verbindung $NH_2-CH_2-CH_2-NH-CH_2-CH_2-NH_2$),

$c_4$) $(Z^2)_2N-CH_2CH_2-N(Z^2)_2$ (IIId)

worin
$Z^2$ = unabhängig voneinander Wasserstoff, 2-Hydroxy-prop-1-yl oder 2-Hydroxy-eth-1-yl, vorzugsweise 2-Hydroxypropyl oder 2-Hydroxyethyl bedeutet,

d) ein Imidazolin-Derivat der Formeln (IVa) oder (IVb)

$d_1$)

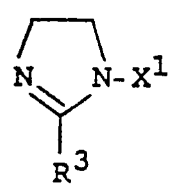

(IVa)

worin
$R^3$ = ein gesättigter oder ein- oder mehrfach ungesättigter aliphatischer Rest mit 7 bis 21 C-Atomen, bevorzugt 12 - 14 oder 16 - 18 C-Atomen,
$X^1$ = Wasserstoff oder $-CO-R^4$ oder Polyoxethylen mit 1 - 55 EO, bevorzugt 4 - 20 EO, Polyoxpropylen mit 1- 40 PyO, bevorzugt 4 - 12 PyO oder ein Blockpolymerisat A und
$R^4$ = $(C_1-C_{22})$Alkyl, bevorzugt $(C_1-C_8)$Alkyl bedeutet,

$d_2$)

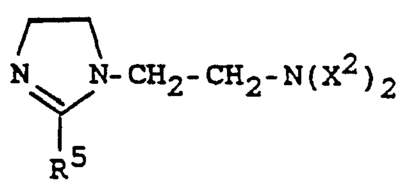

(IVb),

worin
$R^5$ = ein gesättigter oder ein- oder mehrfach ungesättigter aliphatischer Rest mit 6 bis 22 C-Atomen, bevorzugt 12 - 14 C-Atomen, und
$X^2$ = unabhängig voneinander Wasserstoff, Polyoxethylen mit 2 - 55, bevorzugt 2 - 22 EO, Polyoxpropylen mit 1 - 40, bevorzugt 2 - 14 PyO, oder ein Blockpolymerisat A bedeutet.

e) ein Zuckeramin der Formeln (Va) oder (Vb)

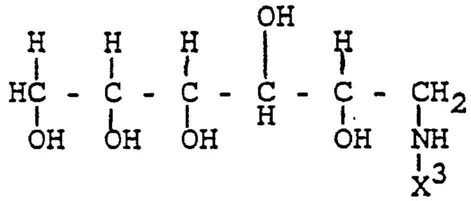

(Va)

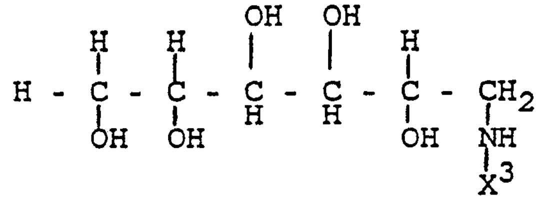

(Vb)

worin
$X^3$ = Wasserstoff, $(C_1\text{-}C_{25})$Alkyl, $(C_2\text{-}C_{25})$Alkenyl oder $-CH_2\text{-}CH_2\text{-}OH$, bevorzugt Wasserstoff, $CH_3$, $C_{12}H_{25}$ oder $CH_2\text{-}CH_2\text{-}OH$
bedeutet,

f) ein Aminoethylethanolamin der Formel (VI)

$(X^4)_2N\text{-}CH_2\text{-}CH_2\text{-}NX^4\text{-}CH_2\text{-}CH_2\text{-}OX^4$ (VI),

worin
$X^4$ = unabhängig voneinander Wasserstoff, Polyoxethylen mit 2 - 55, bevorzugt 4 - 25 EO, Polyoxpropylen mit 2 - 45, bevorzugt 4 - 35 PyO oder ein Blockpolymerisat A bedeutet,

g) ein Alkanolamin der Formel (VII)

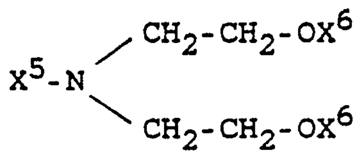

VII

worin
$X^5$ = H, $CH_3$, $CH_2\text{-}CH_3$, $CH_2\text{-}CH_2\text{-}CH_3$, $CH_2\text{-}CH_2\text{-}OX^6$ oder die Bedeutung von $X^6$ und
$X^6$ = unabhängig voneinander Wasserstoff, Polyoxethylen mit 2 - 55 EO, bevorzugt 4- 15 EO, Polyoxpropylen mit 2 - 40 PyO, bevorzugt 2 - 12 PyO oder ein Blockpolymerisat A
bedeutet,

h) eine Verbindung der Formeln (VIIIa), (VIIIb) oder (VIIIc)

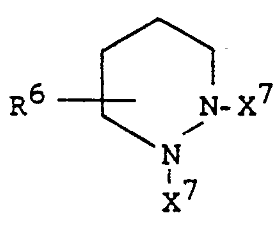

(VIIIa)

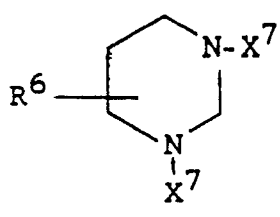

(VIIIb)

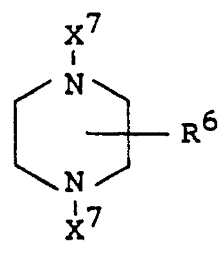

(VIIIc)

worin
$R^6$ = ein gesättigter oder ein- oder mehrfach ungesättigter aliphatischer Rest mit 1 - 22 C-Atomen, bevorzugt 12 - 14 oder 16 - 18 C-Atomen und

$X^7$ = unabhängig voneinander Wasserstoff, Polyoxethylen mit 2 - 60 EO, bevorzugt 8 - 30 EO, Polyoxpropylen mit 2 - 50 PyO, bevorzugt 4 - 20 PyO oder ein Blockpolymerisat A bedeutet,

i) ein Piperidin-Derivat der Formel (IX)

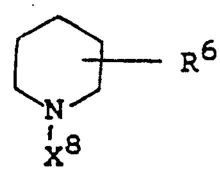

(IX)

worin

$X^8$ = Wasserstoff, Polyoxethylen mit 1- 50 EO, bevorzugt 4 -18 EO, Polyoxpropylen 1 - 40 PyO, bevorzugt 2 - 12 PyO oder ein Blockpolymerisat A bedeutet,

j) ein Morpholin-Derivat der Formel (X)

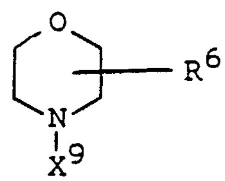

(X) ,

worin

$X^9$ = Wasserstoff, Polyoxethylen mit 1- 65 EO, bevorzugt 4 - 25 EO, Polyoxpropylen mit 1 - 50 PyO, bevorzugt 4 - 15 PyO oder ein Blockpolymerisat A bedeutet, oder die Bedeutung von $R^1$ oder $R^1$-CO- besitzt, oder

k) ein Aminoxid der Formel (XI)

$R^6-\overset{\overset{O}{\uparrow}}{N}(X^{10})_2$ (XI)

worin

$X^{10}$ = $(C_1-C_5)$Alkyl, bevorzugt $(C_1-C_2)$Alkyl, Polyoxethylen mit 1 - 65 EO, bevorzugt 2 - 20 EO, Polyoxpropylen mit 1 - 45 PyO, bevorzugt 2- 15 PyO, oder ein Blockpolymerisat A bedeutet.

Bei den Resten $R^1$, $R^2$, $R^3$, $R^6$ ist der aliphatische Rest, falls er ungesättigt ist, vorzugsweise ein- oder zweifach ungesättigt. Bevorzugt sind jedoch gesättigte aliphatische (=Alkyl)Reste.

Die Polyamine des Typs $c_1$) sind sternförmig konfiguriert in der Weise, daß für m = >0 die mit m indizierten Amino-Reste $(CH_2)_r-N<$ untereinander so verknüpft sind, daß der entstehende Rest verzweigt ist und nur an den Enden Stickstoffatome mit freien Valenzen auftreten, die durch die Reste $-(CH_2)_r-NX_2$ substituiert sind. So ist beispielsweise für m = 4 die Konstitution demnach wie folgt:

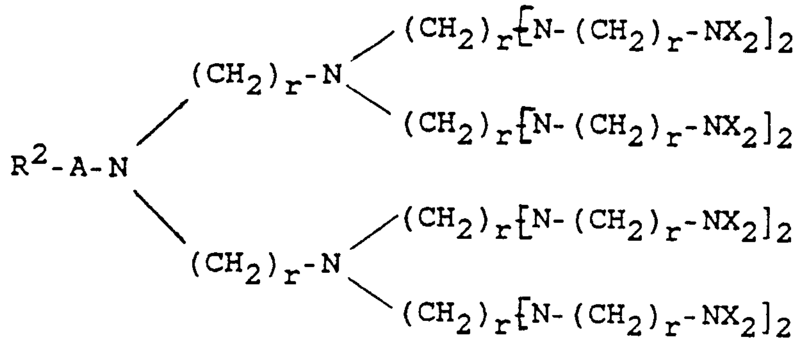

Als Beispiele für die Amine des Typs a) (Formel Ia) sind insbesondere zu nennen: Cocosfettamin, Talgfettamin, als Beispiele für Amine des Typs b) (Formeln IIa) insbesondere Dioctylamin, Didecylamin, Dimethylcocosfettamin, die alle wie oben angegeben ethoxyliert oder propoxyliert, insbesondere ethoxyliert, sein können.

Als Säurekomponenten für die genannten Wirkstoffsalze können beispielsweise eingesetzt werden:

a) substituierte Phenoxycarbonsäuren wie (4-Chlor-2-methylphenoxy)essigsäure (MCPA), 2-(4-Chlor-2-methyl-phenoxy)propionsäure (Mecoprop), 2,4-Dichlorphenoxyessigsäure (2,4-D); 2,4,5-Trichlorphenoxyessigsäure (2,4,5-T) oder 4-(2,4-Dichlorphenoxy)buttersäure (2,4-DB) oder 2-[4-(2,4-Dichlor-phenoxy)-phenoxy]-propionsäure (Diclofop) oder 2-[4-(6-Chlorbenzoxazol-2-yloxy)-phenoxy]-propionsäure (Fenoxaprop);

b) Phosphinsäuren der Formel (XII)

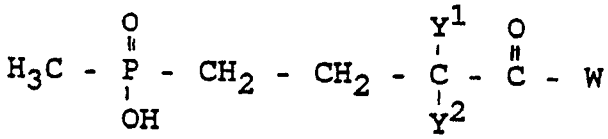

(XII)

worin
$Y^1$ = H,
$Y^2$ = $NH_2$ oder
$Y^1$ und $Y^2$ zusammen = O,
W = OH oder ein Mono- oder Dipeptidrest abgeleitet von den natürlichen Aminosäuren, bevorzugt die Verbindungen der Formel XII mit $Y^1$ = H, $Y^2$ = $NH_2$ und W = OH (XIIa, common name = Glufosinate), $-NHCH(CH_3)-CONHCH(CH_3)-COOH$ (XIIb, common name = Bialaphos) oder $-CH(CH_3)-CONHCH[CH_2CH-(CH_3)_2]COOH$ (XIIc; common name: Phosalacine),

c) die Phosphinylcarbonsäure der Formel (XIII)

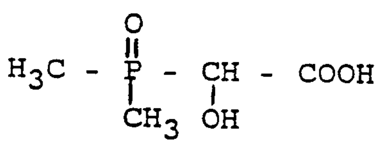

(XIII)

Die genannten Herbizide und einige ihrer Salze sind zum großen Teil in "The Pesticide Manual", 8. Aufl. (1987), British Crop Protection Council beschrieben. Die Verbindungen der Formel XII und bestimmte Salze sind auch aus US-PS 4,168,936; US-PS 4 309 208, DE-OS 35 44 375, J. of Antibiotics Vol. 36(1) S. 96-98 (1983) und Vol. 37(2) S. 542 (1985) bekannt. Die Verbindung der Formel XIII und einige ihrer Salze ist in EP-A 0106114 bzw. US-PS 4 594 098 beschrieben.

Die obengenannten Herbizide können soweit sie optisch aktive Zentren enthalten, z.B. die Verbindungen XIIa - XIIb oder Verbindung XIII als Racemate oder in Form ihrer Enantiomeren eingesetzt werden. Von der Erfindung werden daher sowohl die Racematsalze wie auch die Salze der Enantiomeren oder beliebige Mischungen derselben erfaßt.

Die erfindungsgemäßen Salze besitzen im Vergleich zu den bekannten Salzen wie z.B. dem Ammoniumsalz von Verbindung XIIa oder dem Na-Salz von Verbindung XIII eine deutlich überlegene herbizide Wirkung.

Zur Herstellung der erfindungsgemäßen Salze werden die Herbizid-Säuren mit den Basenkomponenten in wäßrigem Medium umgesetzt. Beispielsweise werden die Herbizid-Säuren in Wasser gelöst oder dispergiert und vorzugsweise bei Temperaturen zwischen 25 und 50 °C mit der obengenannten, gegebenenfalls geeignet gelösten Base oder einem Gemisch derselben tropfenweise versetzt.

Zweckmäßig geschieht dies durch Zutropfen oder Zulaufen der Base unter Temperaturkontrolle.

Es ist nicht erforderlich, äquimolare Mengen der Reaktionspartner einzusetzen, da die erfindungsgemäßen Salze bei ihrer Anwendung in Mischung mit der Herbizid-Säure vorliegen können. Durch Wahl entsprechender Mischungsverhältnisse können sowohl die Ökotoxizität als auch die biologische Wirksamkeit beeinflußt bzw. optimiert werden.

Bei schlechter Löslichkeit der Salze in Wasser ist der Einsatz von Lösevermittlern wie ($C_1$-$C_5$)-Alkanolen, beispielsweise Isopropanol oder Diolen wie Propylenglykol, Ethylenglykol, ferner Polyglykolen oder Dimethylformamid zweckmäßig.

Die vorteilhafte Wirkung der erfindungsgemäßen Salze kann noch durch Zugabe von anionischen oder nichtionischen Netzmitteln oder von Elektrolyten wie beispielsweise Ammoniumchlorid oder Ammoniumsulfat verstärkt werden. Die Netzmittel oder Elektrolyte können entweder in die Lösung bzw. Formulierung der Salze eingearbeitet oder als Tankmix-Zusätze hinzugefügt werden. Ausreichend sind 0,01-0,9 Gew.-% bevorzugt 0,1-0,3 Gew.-% Netzmittel und/oder Elektrolyt bezogen auf die Spritzbrühe.

Als anionische Netzmittel kommen in Frage u.a. Alkali-, Erdalkali- und Ammonium-Salze von Alkyl-aryl-

sulfonsäuren mit einem ($C_6$-$C_{30}$)- bevorzugt ($C_8$-$C_{12}$)Alkyl-Rest, von Alkylsulfaten mit einem ($C_6$-$C_{18}$)Alkyl-, bevorzugt ($C_8$-$C_{14}$)Alkyl-Rest oder von ($C_{12}$-$C_{15}$)Alkylpolyglykolethersulfaten mit 1 - 10, bevorzugt 2 - 5 EO, oder Partialester der mit Alkali, Erdalkali oder Ammoniak ganz oder teilweise neutralisierten Orthophosphorsäuren. Die Partialester können gebildet werden z.B. mit Alkylphenolpolyglykolethern mit einem ($C_6$-$C_{30}$)-, bevorzugt ($C_8$-$C_{12}$)Alkyl-Rest und 2 - 50 EO, bevorzugt 4 - 10 EO, ferner mit Fettalkoholen, Fettalkoholpolyglykolethern mit einem ($C_6$-$C_{22}$)- bevorzugt ($C_{12}$-$C_{14}$)- oder ($C_{16}$-$C_{18}$)-Fettalkoholrest, mit 2,4,6-Tri(1-phenylethyl)phenolpolyglykolethern mit 1 - 60 EO, bevorzugt 4 - 30 EO oder mit anderen Verbindungen.

Als nichtionische Netzmittel bzw. Additive können eingesetzt werden: Alkylphenylpolyglykolether mit einem ($C_6$-$C_{30}$)-bevorzugt ($C_8$-$C_{12}$)-Alkylrest und 2 - 50 EO, bevorzugt 4 - 10 EO, Fettalkoholpolyglykolether mit einem ($C_6$-$C_{22}$)-, bevorzugt, ($C_{12}$-$C_{14}$)- oder ($C_{16}$-$C_{18}$)-Fettalkoholrest mit 1-50, bevorzugt 2 - 10 EO, sowie Derivate dieser Verbindungen, die einen entständigen Methyl- oder Isocyanatrest enthalten, z.B. veretherte Verbindungen der obengenannten nichtionischen Substanzen.

Die Wirkungsverstärkung dieser Netzmittel äußert sich zum Teil überraschenderweise auch in einer Selektivitätssteigerung der betreffenden Salze. So wurde beobachtet, daß monokotyle Pflanzen wie Hordeum vulgare kaum geschädigt werden, dikotyle Pflanzen wie Sinapis alba oder Chrysanthemum segetum aber vollständig kontrolliert werden.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden; bevorzugt ist der Einsatz von wäßrigen Lösungen.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

Die erfindungsgemäßen Mittel enthalten in der Regel 1 bis 95 Gew.-%, vorzugsweise 2 bis 90 Gew.-% der erfindungsgemäßen herbiziden Salze (Wirkstoffe).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten Mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr

Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

In den folgenden Beispielen beziehen sich Prozentangaben auf das Gewicht, sofern nichts anderes angegeben ist.

**Herstellungsbeispiele**

**Allgemeine Vorschrift**

Die Herbizid-Säure wird wie in den Tabellen angegeben in Wasser und/oder einem organischen Lösungsmittel gelöst und bei Temperaturen zwischen 25 - 50 °C mit der betreffenden Base oder deren Gemisch in den gewünschten molaren Mengen tropfenweise versetzt. Nach beendeter Zugabe wird noch 10 Minuten nachgerührt. Man erhält eine leicht gefärbte Lösung. Bei schlechter Löslichkeit der entstandenen Salze ist der Einsatz von Lösevermittlern wie Isopropanol, Propylenglykol, Ethylenglykol oder Polyglykole oder von Dimethylformamid zweckmäßig.

Nach dieser Verfahrensweise lassen sich die Salze der folgenden Beispiele herstellen.

**TABELLE 1**

Beispiele für die Umsetzung der Verbindungen XIIa und XIIb mit Aminkomponenten

Mengen berechnet für 100 g Lösung

| Beispiel Nr. | Aminkomponente | Verbindung XIIa [g] | Aminkomponente [g] | Wasser [g] | Wirkstoffkonzentration [Gew.-%] |
|---|---|---|---|---|---|
| 1 | Cocosfettamin + 2EO | 2,5 | 3,98 | 93,52 | 2,50 % |
| 2 | Cocosfettamin + 5EO | 2,5 | 5,83 | 91,67 | 2,50 % |
| 3 | Cocosfettamin + 10EO | 2,5 | 8,94 | 88,56 | 2,50 % |
| 4 | Cocosfettamin + 20EO | 2,5 | 14,74 | 82,76 | 2,50 % |
| 5 | Talgfettamin + 2EO | 2,5 | 5,17 | 92,33 | 2,50 % |
| 6 | Talgfettamin + 10EO | 2,5 | 9,78 | 87,72 | 2,50 % |
| 7 | Talgfettamin + 15EO | 2,5 | 13,14 | 84,36 | 2,50 % |
| 8 | Dioctylamin + 2EO | 2,5 | 4,59 | 92,91 | 2,50 % |
| 9 | Dioctylamin + 5EO | 2,5 | 6,34 | 91,16 | 2,50 % |
| 10 | Didecylamin + 2EO | 2,5 | 5,30 | 92,20 | 2,50 % |
| 11 | Didecylamin + 5EO | 2,5 | 7.04 | 90,46 | 2,50 % |
| 12 | Dimethylcocosfettamin | 2,5 | 3,25 | 94,25 | 2,50 % |
| 13 | D-Glucamin | 2,5 | 2,51 | 94,99 | 2,50 % |
| 14* | Cocosfettamin + 5EO | 2,5 | 11,66 | 85,84 | 2,50 % |
| 15* | Talgfettamin + 2EO | 2,5 | 10,33 | 87,17 | 2,50 % |

*) Molverhältnis Verbindung XIIa:Amin = 1:2

**TABELLE 1** (Fortsetzung)

| Beispiel Nr. | Aminkomponente | Herbizid-Säure | Aminkomponente [g] | Wasser [g] | Lösungsvermittler u. Netzmittel | Wirkstoffkonzentration [Gew.-%] |
|---|---|---|---|---|---|---|
| 16 | Dodecylamin | 8 g IIa | 8 | 54 | 30 g Dimethylformamid | 8 % |
| 17 | " | 8 g IIa | 8 | 34 | 30 g Dimethylformamid u. 20 g Nonylphenol + 10EO | 8 % |
| 18 | Cocosfettamin + 2EO | 10 g IIa | 22,2 | 50 | 6,7 g Propylenglykolmonomethylether und 11,1 g Natrium-Alkylethersulfat (®Genapol LRO, 70 %ig in Wasser) | 10 % |
| 19 | Talgfettamin + 20EO | 7,8 g IIa | 67,0 | 21,7 | 3,5 g Propylenglykolmonomethylether | 7,8 % |
| 20 | Cocosfettamin + 10EO | 3,2 g IIb | 6,4 | 90,4 | - | 3,2 % |
| 21 | Talgfettamin + 10EO | 3,2 g IIb | 7,2 | 89,6 | - | 3,2 % |
| 22 | Oleylamin + 10EO | 3,2 g IIb | 7,1 | 89,7 | - | 3,2 % |
| 23 | Stearylamin + 10EO | 3,2 g IIb | 7,1 | 89,7 | - | 3,2 % |
| 24 | Talgfettamin + 15EO | 3,2 g IIb | 10 | 86,4 | - | 3,2 % |
| 25 | Talgfettamin + 10EO | 3,2 g IIb | 14,4 | 82,4 | - | 3,2 % |
| 26 | Talgfettamin + 5EO | 2,5 g IIa | 8,2 | 89,3 | - | 2,5 % |
| 27 | Cocosfettamin + 15EO | 2,5 g IIa | 15,0 | 82,5 | - | 2,5 % |

**TABELLE 2**

Beispiele für die Umsetzung der Verbindung XIII mit Aminkomponenten
Mengen berechnet für 100 g Lösung

| Beispiel Nr. | Aminkomponente | Verbindung XIII [g] | m Aminkomponente [g] | m Wasser [g] | Konz. Verbindung XIII [%] |
|---|---|---|---|---|---|
| 1 | Cocosfettamin + 2EO | 2,5 | 4,74 | 92,76 | 2,50 % |
| 2 | Cocosfettamin + 5EO | 2,5 | 6,94 | 90,56 | 2,50 % |
| 3 | Cocosfettamin + 10EO | 2,5 | 10,64 | 86,86 | 2,50 % |
| 4 | Cocosfettamin + 20EO | 2,5 | 17,55 | 79,85 | 2,50 % |
| 5 | Talgfettamin + 2EO | 2,5 | 6,15 | 91,35 | 2,50 % |
| 6 | Talgfettamin + 10 | 2,5 | 11,64 | 85,86 | 2,50 % |
| 7 | Talgfettamin + 15EO | 2,5 | 15,64 | 81,86 | 2,50 % |
| 8 | Dioctylamin + 2EO | 2,5 | 5,46 | 92,04 | 2,50 % |
| 9 | Dioctylamin + 5EO | 2,5 | 7,55 | 89,95 | 2,50 % |
| 10 | Didecylamin + 2EO | 2,5 | 6,32 | 91,18 | 2,50 % |
| 11 | Didecylamin + 5EO | 2,5 | 8,39 | 89,11 | 2,50 % |
| 12 | Dimethylcocosfettamin | 2,5 | 3,87 | 93,63 | 2,50 % |
| 13 | D-Glucamin | 2,5 | 2,99 | 94,51 | 2,50 % |
| 14* | Cocosfettamin + 5EO | 2,5 | 13,88 | 83,62 | 2,50 % |
| 15* | Talgfettamin + 2EO | 2,5 | 12,30 | 85,20 | 2,50 % |

* Molverhältnis Verbindung XIII:Amin = 1:2

**TABELLE 2** (Fortsetzung)

| Beispiel Nr. | Aminkomponente | Verbindung XIII | Wasser [g] | Lösungs- und Netzmittel | Wirkstoff-konzentration |
|---|---|---|---|---|---|
| 16 | 41 g Cocosfettamin + 2EO | 19,3 g | 9,7 g | 20 g Isotridecanol + 6EO und<br>10 g Propylenglykolmonomethylether | 19,3 % |
| 17 | 41 g Cocosfettamin + 2EO | 19,3 g | 7,7 g | 17 g Isotridecanol + 6EO,<br>9 g Polyglykol 200 und<br>6,0 Propylenglykolmonomethylether | 19,3 % |
| 18 | 30 g N,N,N',N'-Tetrakis-(2-hydroxypropyl)-ethylendiamin | 29,6 g | 15,6 g | 8,0 g Propylenglykolmonomethylether und<br>16,8 g einer 40 %igen wäßrigen Lösung eines Na-Sulfobernsteinsäureesters | 29,6 % |
| 19 | 20,2 g N,N,N',N'-Tetrakis-(2-hydroxypropyl)-ethylendiamine | 20,0 g | 19,8 | 10,0 g Propylenglykolmethylether und<br>30,0 g Natrium-Alkylethersulfat (®Genapol LRO, 70 %ig in Wasser) | 20,0 % |
| 20 | 20,2 g N,N,N',N'-Tetrakis-(2-hydroxypropyl)-ethylendiamin und<br>5,0 g Cocosfettamin + 2EO | 20,0 g | 31,5 g | 10,0 g Propylenglykolmonomethylether und<br>13,3 g Isotridecanol + 6EO | 20 % |
| 21 | 40 g Cocosfettamin + 2EO | 20 g | - | 16,8 g Dimethylformamid,<br>10,5 g Nonylphenol + 10EO und<br>12,7 g ®Solvesso 200 | 20 % |
| 22 | 40 g Cocosfettamin + 2EO | 20 g | - | 16,8 g Dimethylformamid,<br>12,7 g ®Solvesso 200 und<br>10,5 g Isotridecanol + 6EO | 20 % |

Beispiel 3.1

EC-Formulierung eines Diclofop-Salzes (EC = wasseremulgierbares Konzentrat)

14.6 g Diclofop werden mit
8,6 g Dodecylamin in
34.28 g Xylol
17 Stunden auf 130° C erwärmt. Nach Abkühlen auf 22° C wird mit
19,05 g Cyclohexanon verdünnt und mit
19.05 g einer Mischung aus einem Calciumsalz einer chlorierten sekundären $C_{13}$-$C_{18}$-Paraffinsulfonsäure und ethoxyliertem Rizinusöl (36 EO) (®Emulsogen ITN) und
4,76 g ethoxylierten 2,4,6-Tributylphenol (30 EO) (®Sapogenat T-300) versetzt.

## Biologische Beispiele

### Beispiel I

Verschiedene Pflanzenarten wurden auf sandigem Lehmboden in Töpfen von 9 cm Durchmesser ausgesät und unter Gewächshausbedingungen angezogen, bis sie ein Stadium von 4 - 5 Blättern erreicht hatten. Dann wurden wie mit den erfindungsgemäßen Salzen besprüht, wobei ein Spritzvolumen von 300 l Wasser ha zur Anwendung kam. Die Pflanzen wurden sodann weitere 3-5 Wochen im Gewächshaus kultiviert. Anschließend wurde die herbizide Wirksamkeit im Vergleich zur unbehandelten Kontrolle visuell bonitiert. Als Vergleichsmittel wurden herkömmliche Formulierungen eingesetzt. Der Versuch wurde mit 2 Wiederholungen durchgeführt.

Pflanzenarten:

SIAL = Sinapis alba
CRSE = Chrysanthemum segetum
AVFA = Avena fatua
AGRE = Agropyron repens
CYDA = Cynodon dactylon

Die Ergebnisse der Versuche sind in Tabelle Ia - Ic dargestellt. Daraus wird die überlegene Wirksamkeit der erfindungsgemäßen Formulierungen gegenüber den Vergleichsformulierungen sichtbar.

Tabelle Ia

| Herbizide Wirkung verschiedener erfindungsgemäßer Salze enthaltend Wirkstoff der Formel XIII (Bonitur 5 Wochen nach Applikation) | | | |
|---|---|---|---|
| Salz | Dosis in kg AS/ha | Herbizide Wirksamkeit in % | |
| | | AGRE | CYDA |
| gemäß Bsp.1, Tab.2 | 2.0<br>1.0<br>0.5 | 92<br>80<br>65 | 80<br>65<br>60 |
| gemäß Bsp.2, Tab.2 | 2.0<br>1.0<br>0.5 | 95<br>85<br>65 | 90<br>70<br>65 |
| gemäß Bsp.5, Tab.2 | 2.0<br>1.0<br>0.5 | 95<br>90<br>60 | 90<br>65<br>50 |
| gemäß Bsp.6, Tab.2 | 2.0<br>1.0<br>0.5 | 95<br>92<br>85 | 90<br>75<br>55 |
| gemäß Bsp.7, Tab.2 | 2.0<br>1.0<br>0.5 | 92<br>85<br>60 | 90<br>75<br>60 |
| Vergleichs: Na-Salz | 2.0<br>1.0<br>0.5 | 85<br>60<br>50 | 40<br>35<br>25 |
| AS bedeutet hier wie in den folgenden Tabellen die eingesetzte Menge der Herbizid-Säure des betreffenden Salzes. | | | |

Tabelle Ib

| Herbizide Wirkung verschiedener Salze der Verbindung XIIb (Bialaphos) (Bonitur 3 Wochen nach Behandlung) | | | |
|---|---|---|---|
| Salz | Dosis in kg AS/ha | Herbizide Wirksamkeit in % | |
| | | AGRE | CYDA |
| gemäß Bsp. 20, Tab. 1 | 0.8<br>0.4<br>0.2 | 100<br>85<br>70 | 98<br>90<br>65 |
| gemäß Bsp. 21, Tab. 1 | 0.8<br>0.4<br>0.2 | 100<br>80<br>70 | 95<br>80<br>50 |
| gemäß Bsp. 24, Tab. 1 | 0.8<br>0.4<br>0.2 | 100<br>90<br>80 | 92<br>85<br>60 |
| gemäß Bsp. 22, Tab. 1 | 0.8<br>0.4<br>0.2 | 100<br>92<br>75 | 95<br>90<br>75 |
| gemäß Bsp. 23, Tab. 1 | 0.8<br>0.4<br>0.2 | 99<br>90<br>70 | 98<br>95<br>60 |
| Vergleich: Na-Salz | 0.8<br>0.4<br>0.2 | 95<br>70<br>40 | 90<br>50<br>30 |

Tabelle Ic: Herbizide Wirksamkeit verschiedener erfindungsgemäßer Salze von Glufosinate (Verbindung XIIa) bei Zusatz von Additiven (Bonitur 3 Wochen nach Applikation)

| Salz | Dosis in kg AS/ha | Herbizide Wirksamkeit in % SIAL | CHSE | AVFA |
|---|---|---|---|---|
| gemäß Bsp.2, Tab.1 | 0.5 | 100 | 100 | 95 |
| +X-060 | 0.25 | 98 | 98 | 85 |
| | 0.12 | 60 | 96 | 40 |
| gemäß Bsp.6, Tab.1 | 0.5 | 100 | 100 | 90 |
| +X-060 | 0.25 | 99 | 100 | 75 |
| | 0.12 | 95 | 98 | 40 |
| gemäß Bsp.7, Tab.1 | 0.5 | 100 | 100 | 85 |
| +X-060 | 0.25 | 97 | 100 | 60 |
| | 0.12 | 90 | 95 | 45 |
| gemäß Bsp.2, Tab.1 | 0.5 | 100 | 100 | 90 |
| +N-060 | 0.25 | 99 | 95 | 75 |
| | 0.12 | 84 | 90 | 40 |
| gemäß Bsp.6, Tab.1 | 0.5 | 100 | 100 | 95 |
| +N-060 | 0.25 | 99 | 100 | 70 |
| | 0.12 | 80 | 85 | 40 |
| gemäß Bsp.7, Tab.1 | 0.5 | 100 | 100 | 90 |
| +N-060 | 0.25 | 100 | 98 | 65 |
| | 0.12 | 85 | 80 | 40 |
| gemäß Bsp.2, Tab.1 | 0.5 | 100 | 100 | 80 |
| +LRO | 0.25 | 100 | 100 | 70 |
| | 0.12 | 92 | 98 | 40 |

| Salz | Dosis in kg AS/ha | Herbizide Wirksamkeit in % SIAL | CHSE | AVFA |
|---|---|---|---|---|
| gemäß Bsp.6, Tab.1 | 0.5 | 100 | 100 | 85 |
| +LRO | 0.25 | 95 | 100 | 75 |
| | 0.12 | 95 | 100 | 40 |
| gemäß Bsp. 7, Tab.1 | 0.5 | 100 | 100 | 80 |
| +LRO | 0.25 | 98 | 100 | 70 |
| | 0.12 | 95 | 99 | 30 |
| gemäß Bsp.2, Tab.1 | 0.5 | 100 | 100 | 95 |
| +Laurylsulfat | 0.25 | 100 | 100 | 55 |
| | 0.12 | 90 | 97 | 40 |
| Vergleich: | 0.5 | 93 | 99 | 60 |
| $NH_4$-Salz | 0.25 | 60 | 70 | 35 |
| | 0.12 | 30 | 45 | 10 |

X-060 = Isotridecanol + 5 Mol EO
N-060 = Nonylphenol + 5 Mol EO
LRO = Laurylpolyglykolethersulfatnatriumsalz

Die Konzentration der Additive X-060, N-060 und LRO beträgt jeweils 0,3 % bezogen auf das Spritzvolumen von 300 l/ha.

Beispiel II

In Töpfen von 13 cm Durchmesser wurden verschiedene Pflanzenarten auf sandigem Lehmboden ausgesät und anschließend bei guten Wachstumsbedingungen bis zur Größe von 4-6 Blättern unter Freilandbedingungen angezogen. Dann wurden sie mit den verschiedenen erfindungsgemäßen Salzen der beanspruchten Herbizidwirkstoffe behandelt. Hierbei kam ein Sprühvolumen von 300 l/ha Wasser zur Anwendung. Die Pflanzen wurden nach der Herbizidbehandlung weitere 5 Wochen unter guten Wachstumsbedingungen im Freien kultiviert, und anschließend wurde die herbizide Wirkung im Vergleich zu unbehandelten Kontrollpflanzen visuell bonitiert, wobei die Schädigung der Pflanzen in Prozent geschätzt wurde. Es wurden zwei Wiederholungen durchgeführt.

Pflanzenarten:

SIAL = Sinapis alba
CRSE = Chrysanthemum segetum

HOVU = Hordeum vulgare
AGRE = Agropyron repens
CYDA = Cynodon dactylon

Die Ergebnisse der Bonituren sind in Tabelle IIa dargestellt. Sie zeigen die überlegene herbizide Wirkung der erfindungsgemäßen Salze. Darüber hinaus wirken einige Salze gegen grasartige Kulturpflanzen wie z.B. Gerste deutlich schwächer, als gegen breitblättrige, so daß sie selektiv in Getreidearten zur Bekämpfung von Unkräutern eingesetzt werden können.

Tabelle IIa: Herbizide Wirksamkeit und Selektivität verschiedener Salze von Glufosinate (Verbindung XIIa)

| Salz | Dosis in kg AS | Herbizide Wirksamkeit in % SIAL | CRSE | HOVU |
|---|---|---|---|---|
| gemäß Bsp.3, Tab.1 | 1.0 | 100 | 100 | 60 |
| | 0.5 | 95 | 100 | 40 |
| | 0.25 | 60 | 85 | 20 |
| gemäß Bsp. 27, Tab. 1 | 1.0 | 100 | 100 | 65 |
| | 0.5 | 90 | 98 | 25 |
| | 0.25 | 50 | 80 | 5 |

| Salz | Dosis in kg AS | Herbizide Wirksamkeit in % SIAL | CRSE | HOVU |
|---|---|---|---|---|
| gemäß Bsp. 26, Tab. 1 | 1.0 | 100 | 100 | 65 |
| | 0.5 | 95 | 100 | 15 |
| | 0.25 | 85 | 90 | 5 |
| gemäß Bsp.7, Tab.1 | 1.0 | 100 | 100 | 45 |
| | 0.5 | 98 | 100 | 20 |
| | 0.25 | 90 | 95 | 0 |
| Vergleich: $NH_4$-Salz | 1.0 | 85 | 100 | 65 |
| | 0.5 | 65 | 85 | 30 |
| | 0.25 | 20 | 40 | 10 |

## Ansprüche

1. Salze von Herbizid-Säuren aus der Gruppe, bestehend aus substituierten Phenoxycarbonsäuren, Phosphinsäuren der Formel

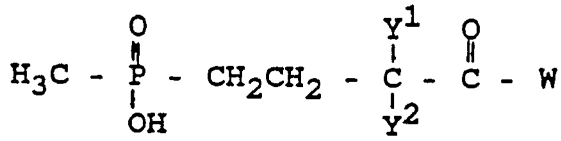

worin
$Y^1$ Wasserstoff und $Y^2$ Amino oder $Y^1$ und $Y^2$ zusammen ein Sauerstoffatom bedeuten und
W Hydroxy oder ein von natürlichen Aminosäuren abgeleiteter Mono- oder Dipeptidrest ist, und einer Phosphinylcarbonsäure der Formel

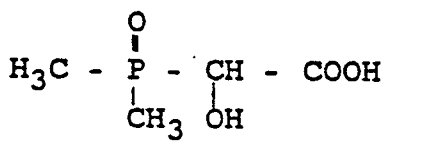
,

dadurch gekennzeichnet, daß sie als Basenkomponente enthalten:

a) ein primäres Fettamin oder Fettamid der Formeln (Ia) oder (Ib)

$$R^1 - NX_2$$

(Ia)

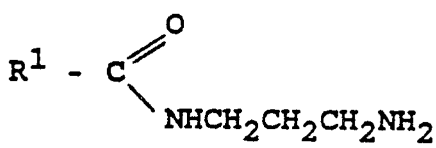
,

(Ib)

worin
$R^1$ = ein gesättigter oder ein- oder mehrfach ungesättigter aliphatischer Rest mit 6 - 22 C-Atomen; Alkyl- oder Alkenylphenyl, Alkyl- oder Alkenylcyclohexyl, wobei der Alkyl- oder Alkenylrest jeweils 1 - 30 C-Atome, bevorzugt 8 - 12 C-Atome enthält, und
X = Wasserstoff, Polyoxethylen mit 1 - 40 Mol EO Polyoxypropylen mit 1 - 45 Mol PyO oder ein Polyoxypropylen/Polyoxethylen-Blockcopolymerisat ("Blockpolymerisat A"), mit einem Molverhältnis PyO/EO von 1:9 bis 9:1
bedeutet;

b) ein sekundäres oder tertiäres Amin der Formeln (IIa), (IIb) oder (IIc),

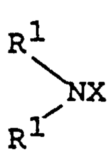

(IIa)

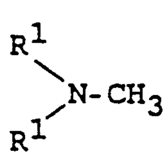

(IIb)

$$R^1-N(CH_3)_2$$

(IIc)

worin
$R^1$ die obengenannte Bedeutung besitzt,

c) ein Polyamin der nachfolgend unter $c_1$) - $c_4$) definierten Formeln (IIIa), (IIIb), (IIIc) oder (IIId)

$c_1$) $R^2$-A-N[$(CH_2)_r$-N$<$]$_m$ [$(CH_2)_r$-$NX_2$]$_n$ (IIIa)

worin
$R^2$ = ein gesättigter oder ein- oder mehrfach ungesättigter aliphatischer Rest mit 1 - 21 C-Atomen,

A = -CO- oder $-CH_2-$
m = die Zahl 0, 2, 6 oder 14
n = m + 2, und
r = 2 oder 3, bedeutet,

$c_2$) $R^2-A-NZ^1-[(CH_2)_r(NZ^1)]_s-(CH_2)_r N(Z^1)_2$ (IIIb)

worin
s = eine ganze Zahl von 1 - 20
$Z^1$ = Wasserstoff oder Polyoxethylen mit 4 - 70 EO Polyoxpropylen mit 4 - 60 PyO oder ein Blockpolymerisat A, bedeutet,

$c_3$) $(Z^2)_2N-[(CH_2)_p-NZ^2]_o-(CH_2)_p-N(Z^2)_2$ (IIIc)

worin
o = eine ganze Zahl von O bis 4,
p = unabhängig voneinander eine ganze Zahl von 2 bis 6,
$Z^2$ = Wasserstoff, Polyoxethylen mit 5 - 70 EO, Polyoxpropylen mit 4 - 60 PyO, oder ein Blockpolymerisat A bedeutet,

$c_4$) $(Z^2)_2N-CH_2CH_2-N(Z^2)_2$ (IIId)

worin
$Z^2$ = unabhängig voneinander Wasserstoff, 2-Hydroxy-prop-1-yl oder 2-Hydroxy-eth-1-yl, vorzugsweise 2-Hydroxypropyl oder 2-Hydroxyethyl bedeutet,

d) ein Imidazolin-Derivat der Formeln (IVa) oder (IVb)

$d_1$)

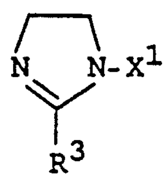

(IVa)

worin
$R^3$ = ein gesättigter oder ein- oder mehrfach ungesättigter aliphatischer Rest mit 7 bis 21 C-Atomen,
$X^1$ = Wasserstoff oder $-CO-R^4$ oder Polyoxethylen mit 1 - 55 EO, bevorzugt 4 - 20 EO, Polyoxpropylen mit 1- 40 PyO, bevorzugt 4 - 12 PyO oder ein Blockpolymerisat A und
$R^4$ = $(C_1-C_{22})$Alkyl, bevorzugt $(C_1-C_8)$Alkyl bedeutet,

$d_2$)

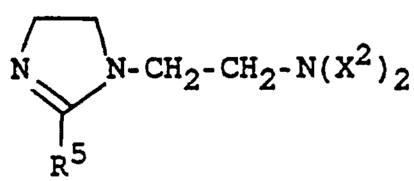

(IVb),

worin
$R^5$ = ein gesättigter oder ein- oder mehrfach ungesättigter aliphatischer Rest mit 6 bis 22 C-Atomen und
$X^2$ = unabhängig voneinander Wasserstoff, Polyoxethylen mit 2 - 55, Polyoxpropylen mit 1 - 40, oder ein Blockpolymerisat A
bedeutet,

e) ein Zuckeramin der Formeln (Va) oder (Vb)

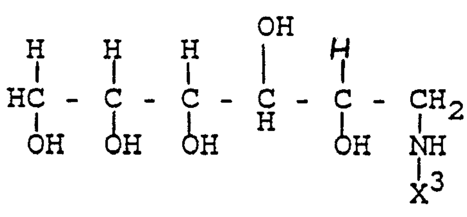

(Va)

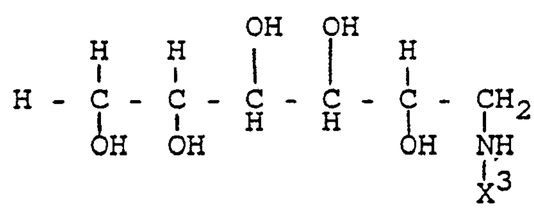

(Vb)

worin

$X^3$ = Wasserstoff, $(C_1-C_{25})$Alkyl, $(C_2-C_{25})$Alkenyl oder $-CH_2-CH_2-OH$,

bedeutet,

f) ein Aminoethylethanolamin der Formel (VI)

$(X^4)_2N-CH_2-CH_2-NX^4-CH_2-CH_2-OX^4$ (VI),

worin

$X^4$ = unabhängig voneinander Wasserstoff, Polyoxethylen mit 2 - 55, Polyoxypropylen mit 2 - 45, oder ein Blockpolymerisat A

bedeutet,

g) ein Alkanolamin der Formel (VII)

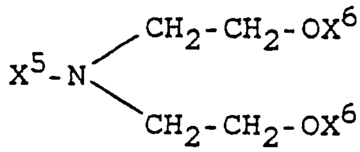

VII

worin

$X^5$ = H, $CH_3$, $CH_2-CH_3$, $CH_2-CH_2-CH_3$, $CH_2-CH_2-OX^6$ oder die Bedeutung von $X^6$ und

$X^6$ = unabhängig voneinander Wasserstoff, Polyoxethylen mit 2 - 55 EO, Polyoxpropylen mit 2 - 40 PyO, oder ein Blockpolymerisat A

bedeutet,

h) eine Verbindung der Formeln (VIIIa), (VIIIb) oder (VIIIc)

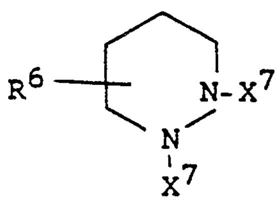

(VIIIa)

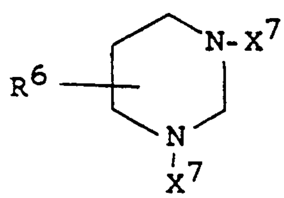

(VIIIb)

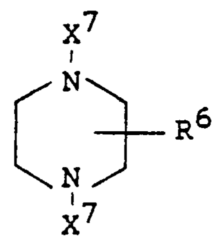

(VIIIc)

worin

$R^6$ = ein gesättigter oder ein- oder mehrfach ungesättigter aliphatischer Rest mit 1 - 22 C-Atomen, und

$X^7$ = unabhängig voneinander Wasserstoff, Polyoxethylen mit 2 - 60 EO, Polyoxpropylen mit 2 - 50 PyO, oder ein Blockpolymerisat A

bedeutet,

i) ein Piperidin-Derivat der Formel (IX)

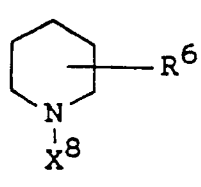

worin

$X^8$ = Wasserstoff, Polyoxethylen mit 1- 50 EO, Polyoxpropylen 1 - 40 PyO, oder ein Blockpolymerisat A und

$R^6$ = den unter h) definierten Rest $R^6$

bedeutet,

j) ein Morpholin-Derivat der Formel (X)

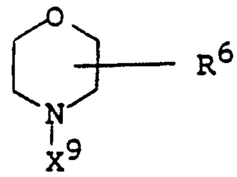

(X) ,

worin
$R^6$ = den unter h) definierten Rest $R^6$ und
$X^9$ = Wasserstoff, Polyoxethylen mit 1- 65 EO, Polyoxpropylen mit 1 - 50 PyO oder ein Blockpolymerisat A bedeutet, oder die Bedeutung von $R^1$ oder $R^1$-CO-besitzt, oder

k) ein Aminoxid der Formel (XI)

O
↑
$R^6$- N $(X^{10})_2$ (XI)

worin
$R^6$ = den unter h) definierten Rest $R^6$ und
$X^{10}$ = $(C_1\text{-}C_5)$Alkyl, Polyoxethylen mit 1 - 65 EO, Polyoxpropylen mit 1 - 45 PyO oder ein Blockpolymerisat A bedeutet.

2. Salze gemäß Anspruch 1, worin die Basenkomponente ein primäres Fettamin der Formel Ia oder ein sekundäres Amin der Formel IIa bedeutet.

3. Salze gemäß Anspruch 1 oder 2, worin $R^1$ ein gesättigter oder ein- oder mehrfach ungesättigter aliphatischer Rest mit 12 bis 18 C-Atomen und X Wasserstoff, Polyoxethylen mit 2 bis 20 Mol EO, insbesondere 2 bis 15 Mol EO, Polyoxpropylen mit 2 bis 15 Mol PyO oder ein Polyoxypropylen/Polyoxethylen-Blockpolymerisat mit einem Molverhältnis PyO/EO von 1:9 bis 9:1 bedeuten.

4. Salze gemäß einem oder mehreren der Ansprüche 1 bis 3, worin die Basenkomponente ein vorzugsweise ethoxyliertes Dodecylamin, Oleylamin, Stearylamin, Cocosfettamin, Talgfettamin, Dioctylamin, Didecylamin oder Dimethylcocosfettamin bedeutet.

5. Salze gemäß einem oder mehreren der Ansprüche 1 bis 4, dadurch gekennzeichnet, daß die Herbizid-Säure eine Verbindung der folgenden Gruppen a) bis c) ist:

a) eine substituierte Phenoxycarbonsäure aus der Gruppe aus MCPA, Mecoprop, 2,4-D, 2,4,5-T, 2,4-DB, Diclofop und Fenoxaprop,

b) eine Phosphinsäure aus der Gruppe Glufosinate, Bialaphos und Phosalacine,

c) 2-Dimethylphosphinoyl-2-hydroxy-essigsäure,

6. Herbizide Mittel, dadurch gekennzeichnet, daß sie ein Salz gemäß einem oder mehreren der Ansprüche 1 bis 5 neben inerten Hilfsstoffen enthalten.

7. Herbizide Mittel gemäß Anspruch 6, dadurch gekennzeichnet, daß als inerter Hilfsstoff Wasser eingesetzt ist.

8. Herbizide Mittel gemäß Ansprüchen 6 oder 7, dadurch gekennzeichnet, daß als inerte Hilfsstoffe anionische oder nichtionische Netzmittel oder Elektrolyte enthalten sind.

9. Verwendung von Salzen gemäß einem oder mehreren der Ansprüche 1 bis 5 als Herbizide.

10. Verfahren zur Bekämpfung von unerwünschtem Pflanzenwuchs, dadurch gekennzeichnet, daß man auf die Pflanzen oder die Anbauflächen eine wirksame Menge eines Salzes gemäß einem oder mehreren der Ansprüche 1 bis 5 appliziert.

11. Verfahren zur Herstellung eines herbiziden Salzes nach einem oder mehreren der Ansprüche 1 bis 5, dadurch gekennzeichnet, daß man die als Säurekomponente zu verwendende Herbizid-Säure mit der Basenkomponente in wäßrigem Medium umsetzt.

Patentansprüche für folgenden Vertragsstaat: ES

1. Verwendung von Salzen, welche als Säurekomponente Herbizid-Säuren aus der Gruppe, bestehend aus substituierten Phenoxycarbonsäuren, Phosphinsäuren der Formel

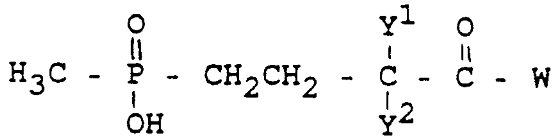

worin
Y' Wasserstoff und $Y^2$ Amino oder Y' und $Y^2$ zusammen ein Sauerstoffatom bedeuten und
W Hydroxy oder ein von natürlichen Aminosäuren abgeleiteter Mono- oder Dipeptidrest ist, und einer Phosphinylcarbonsäure der Formel

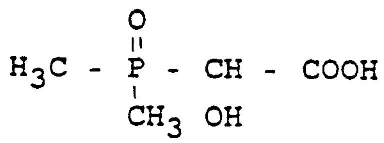
,

und als Basenkomponente

a) ein primäres Fettamin oder Fettamid der Formeln (Ia) oder (Ib)

$R^1 - NX_2$

(Ia)

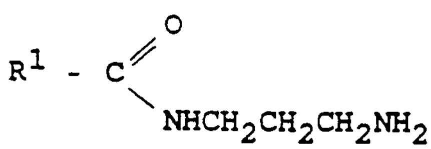
,

(Ib)

worin
R' = ein gesättigter oder ein- oder mehrfach ungesättigter aliphatischer Rest mit 6 - 22 C-Atomen; Alkyl- oder Alkenylphenyl, Alkyl- oder Alkenylcyclohexyl, wobei der Alkyl- oder Alkenylrest jeweils 1 - 30 C-Atome, bevorzugt 8 - 12 C-Atome enthält, und
X = Wasserstoff, Polyoxethylen mit 1 - 40 Mol EO Polyoxypropylen mit 1 - 45 Mol PyO oder ein Polyoxypropylen/Polyoxethylen-Blockcopolymerisat ("Blockpolymerisat A"), mit einem Molverhältnis PyO/EO von 1:9 bis 9:1
bedeutet;

b) ein sekundäres oder tertiäres Amin der Formeln (IIa), (IIb) oder (IIc),

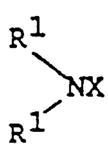

(IIa)

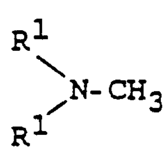

(IIb)

$R^1\text{-N}(CH_3)_2$

(IIc)

worin
R' die obengenannte Bedeutung besitzt,

c) ein Polyamin der nachfolgend unter $c_1$) - $c_4$) definierten Formeln (IIIa), (IIIb), (IIIc) oder (IIId)

$c_1$) $R^2\text{-A-N}[(CH_2)_r\text{-N}<]_m\,[(CH_2)_r\text{-NX}_2]_n$ (IIIa)

worin
$R^2$ = ein gesättigter oder ein- oder mehrfach ungesättigter aliphatischer Rest mit 1 - 21 C-Atomen,
A = -CO- oder $-CH_2-$
m = die Zahl 0, 2, 6 oder 14
n = m + 2, und
r = 2 oder 3, bedeutet,

$c_2$) $R^2\text{-A-NZ}^1\text{-}[(CH_2)_r(NZ^1)]_s\text{-}(CH_2)_r\,N(Z^1)_2$ (IIIb)

worin
s = eine ganze Zahl von 1 - 20
$Z^1$ = Wasserstoff oder Polyoxethylen mit 4 - 70 EO Polyoxpropylen mit 4 - 60 PyO oder ein Blockpolymerisat A, bedeutet,

$c_3$) $(Z^2)_2N-[(CH_2)_p-NZ^2]_o-(CH_2)_p-N(Z^2)_2$ (IIIc)

worin
o = eine ganze Zahl von O bis 4,
p = unabhängig voneinander eine ganze Zahl von 2 bis 6,
$Z^2$ = Wasserstoff, Polyoxethylen mit 5 - 70 EO, Polyoxpropylen mit 4 - 60 PyO, oder ein Blockpolymerisat A bedeutet,

$c_4$) $(Z^2)_2N-CH_2CH_2-N(Z^2)_2$ (IIId)

worin
$Z^2$ = unabhängig voneinander Wasserstoff, 2-Hydroxy-prop-1-yl oder 2-Hydroxy-eth-1-yl, vorzugsweise 2-Hydroxypropyl oder 2-Hydroxyethyl bedeutet,

d) ein Imidazolin-Derivat der Formeln (IVa) oder (IVb)

$d_1$) 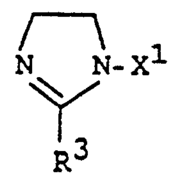 (IVa)

worin
$R^3$ = ein gesättigter oder ein- oder mehrfach ungesättigter aliphatischer Rest mit 7 bis 21 C-Atomen,
$X^1$ = Wasserstoff oder $-CO-R^4$ oder Polyoxethylen mit 1 - 55 EO, bevorzugt 4 - 20 EO, Polyoxpropylen mit 1- 40 PyO, bevorzugt 4 - 12 PyO oder ein Blockpolymerisat A und
$R^4$ = $(C_1-C_{22})$Alkyl, bevorzugt $(C_1-C_8)$Alkyl bedeutet,

$d_2$) 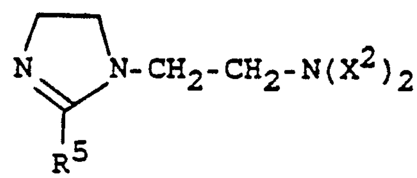 (IVb),

worin
$R^5$ = ein gesättigter oder ein- oder mehrfach ungesättigter aliphatischer Rest mit 6 bis 22 C-Atomen und
$X^2$ = unabhängig voneinander Wasserstoff, Polyoxethylen mit 2 - 55, Polyoxpropylen mit 1 - 40, oder ein Blockpolymerisat A
bedeutet,

e) ein Zuckeramin der Formeln (Va) oder (Vb)

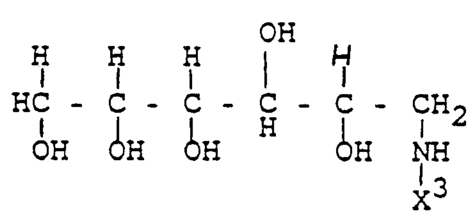

(Va)

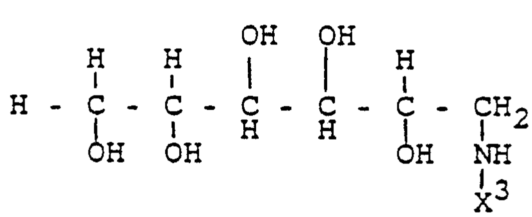

(Vb)

worin
$X^3$ = Wasserstoff, $(C_1-C_{25})$Alkyl, $(C_2-C_{25})$Alkenyl oder $-CH_2-CH_2-OH$,
bedeutet,

f) ein Aminoethylethanolamin der Formel (VI)

$(X^4)_2N-CH_2-CH_2-NX^4-CH_2-CH_2-OX^4$ (VI),

worin

$X^4$ = unabhängig voneinander Wasserstoff, Polyoxethylen mit 2 - 55, Polyoxypropylen mit 2 - 45, oder ein Blockpolymerisat A

bedeutet,

g) ein Alkanolamin der Formel (VII)

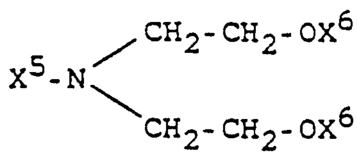

VII

worin

$X^5$ = H, $CH_3$, $CH_2$-$CH_3$, $CH_2$-$CH_2$-$CH_3$, $CH_2$-$CH_2$-$OX^6$ oder die Bedeutung von $X^6$ und

$X^6$ = unabhängig voneinander Wasserstoff, Polyoxethylen mit 2 - 55 EO, Polyoxpropylen mit 2 - 40 PyO, oder ein Blockpolymerisat A

bedeutet,

h) eine Verbindung der Formeln (VIIIa), (VIIIb) oder (VIIIc)

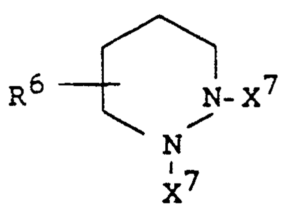

(VIIIa)

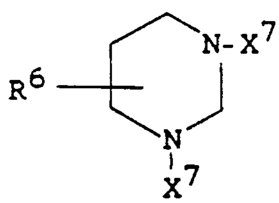

(VIIIb)

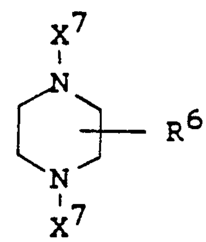

(VIIIc)

worin

$R^6$ = ein gesättigter oder ein- oder mehrfach ungesättigter aliphatischer Rest mit 1 - 22 C-Atomen, und

$X^7$ = unabhängig voneinander Wasserstoff, Polyoxethylen mit 2 - 60 EO, Polyoxpropylen mit 2 - 50 PyO, oder ein Blockpolymerisat A

bedeutet,

i) ein Piperidin-Derivat der Formel (IX)

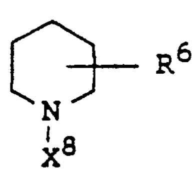

worin

$X^8$ = Wasserstoff, Polyoxethylen mit 1- 50 EO, Polyoxpropylen 1 - 40 PyO, oder ein Blockpolymerisat A und

$R^6$ = den unter h) definierten Rest $R^6$

bedeutet,

j) ein Morpholin-Derivat der Formel (X)

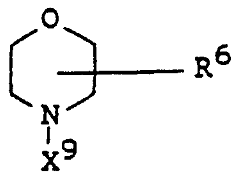

(X) ,

worin

$R^6$ = den unter h) definierten Rest $R^6$ und

$X^9$ = Wasserstoff, Polyoxethylen mit 1- 65 EO, Polyoxpropylen mit 1 - 50 PyO oder ein Blockpolymerisat A bedeutet, oder die Bedeutung von $R^1$ oder $R^1$-CO-besitzt, oder

k) ein Aminoxid der Formel (XI)

$R^6-\overset{\overset{O}{\uparrow}}{N}(X^{10})_2$ (XI)

worin

$R^6$ = den unter h) definierten Rest $R^6$ und

$X^{10}$ = $(C_1$-$C_5)$Alkyl, Polyoxethylen mit 1 - 65 EO, Polyoxpropylen mit 1 - 45 PyO oder ein Blockpolymerisat A bedeutet, enthalten, als Herbizide.

2. Verwendung gemäß Anspruch 1, worin die Basenkomponente ein primäres Fettamin der Formel Ia oder ein sekundäres Amin der Formel IIa bedeutet.

3. Verwendung gemäß Anspruch 1 oder 2, worin $R^1$ ein gesättigter oder ein- oder mehrfach ungesättigter aliphatischer Rest mit 12 bis 18 C-Atomen und X Wasserstoff, Polyoxethylen mit 2 bis 20 Mol EO, insbesondere 2 bis 15 Mol EO, Polyoxpropylen mit 2 bis 15 Mol PyO oder ein Polyoxypropylen/Polyoxethylen-Blockpolymerisat mit einem Molverhältnis PyO/EO von 1:9 bis 9:1 bedeuten.

4. Verwendung gemäß einem oder mehreren der Ansprüche 1 bis 3, worin die Basenkomponente ein vorzugsweise ethoxyliertes Dodecylamin, Oleylamin, Stearylamin, Cocosfettamin, Talgfettamin, Dioctylamin, Didecylamin oder Dimethylcocosfettamin bedeutet.

5. Verwendung gemäß einem oder mehreren der Ansprüche 1 bis 4, dadurch gekennzeichnet, daß die Herbizid-Säure eine Verbindung der folgenden Gruppen a) bis c) ist:

a) eine substituierte Phenoxycarbonsäure aus der Gruppe aus MCPA, Mecoprop, 2,4-D, 2,4,5-T, 2,4-DB, Diclofop und Fenoxaprop,

b) eine Phosphinsäure aus der Gruppe Glufosinate, Bialaphos und Phosalacine,

c) 2-Dimethylphosphinoyl-2-hydroxy-essigsäure,

6. Verwendung gemäß gemäß einem oder mehreren der Ansprüche 1 bis 5, dadurch gekennzeichnet, daß die Salze zusammen mit inerten Hilfsstoffen verwendet werden.

7. Verwendung gemäß Anspruch 6, dadurch gekennzeichnet, daß als inerter Hilfsstoff Wasser eingesetzt ist.

8. Verwendung gemäß Ansprüchen 6 oder 7, dadurch gekennzeichnet, daß als inerte Hilfsstoffe anionische oder nichtionische Netzmittel oder Elektrolyte enthalten sind.

9. Verfahren zur Bekämpfung von unerwünschtem Pflanzenwuchs, dadurch gekennzeichnet, daß man auf die Pflanzen oder die Anbauflächen eine wirksame Menge eines nach einem oder mehreren der Ansprüche 1 bis 5 definierten Salzes appliziert.

10. Verfahren zur Herstellung der in nach einem oder mehreren der Ansprüche 1 bis 5 definierten Salze, dadurch gekennzeichnet, daß man die als Säurekomponente zu verwendende Herbizid-Säure mit der Basenkomponente in wäßrigem Medium umsetzt.